# EUROPÄISCHE PATENTSCHRIFT

(11) **EP 4 294 468 B1**
(45) Veröffentlichungstag und Bekanntmachung des Hinweises auf die Patenterteilung: **08.10.2025**
(21) Anmeldenummer: 22712494.8
(22) Anmeldetag: 15.02.2022
(51) Int. Cl.: A61L 27/00

(54) **IMPLANTAT ZUM IMPLANTIEREN IN EINEN ORGANISMUS UND VERFAHREN**
IMPLANT FOR IMPLANTING IN AN ORGANISM AND PROCEDURE
IMPLANT POUR IMPLANTATION DANS UN ORGANISME ET PROCEDURE

(30) Priorität: 17.02.2021 DE 102021103786
(43) Veröffentlichungstag der Anmeldung: 27.12.2023
(73) Patentinhaber: Fraunhofer-Gesellschaft zur Förderung der angewandten Forschung e.V., 80686 München (DE); Helmholtz-Zentrum hereon GmbH, 21502 Geesthacht (DE)
(72) Erfinder: JANZEN, Kevin, 21029 Hamburg (DE); BURESCH, Hendrik, 21502 Geesthacht (DE); EBEL, Thomas, 21502 Geesthacht (DE); IMGRUND, Philipp, 21029 Hamburg (DE)
(74) Vertreter: Heeschen Pültz Patentanwälte PartGmbB
(86) Internationale Anmeldenummer: PCT/DE2022/100127
(87) Internationale Veröffentlichungsnummer: WO 2022/174868

(56) Entgegenhaltungen:
- WO-A1-2019/072736
- WO-A2-2009/127423
- DE-A1- 102005 033 101
- DE-A1- 102016 011 717
- US-A1- 2009 081 313
- US-A1- 2010 076 544
- US-B2- 7 879 367

## Beschreibung

Die Erfindung betrifft ein Implantat zum Implantieren in einen Organismus, insbesondere in den menschlichen Körper, und ein Verfahren zur Herstellung eines Implantats.

Implantate sind grundsätzlich bekannt. Ein Implantat ist ein in einen Organismus einzusetzendes, künstliches Material, das permanent oder temporär für einen definierten Zeitraum im Organismus verbleibt. Implantate haben häufig die Aufgabe, Körperfunktionen zu unterstützen oder zu ersetzen. Implantate werden beispielsweise aus Titan oder Kunststoff hergestellt.

Medizinische Implantate können eine permanente, dauerhafte Funktion im Organismus übernehmen oder im Organismus temporär, für eine vordefinierte Zeit diese Funktion übernehmen. Die zuletzt genannten Implantate werden üblicherweise wieder aus dem Organismus entfernt. Dies ist unter anderem erforderlich, wenn sich der Organismus in einer Wachstumsphase befindet, wie beispielsweise bei Kindern und jungen Erwachsenen, und das eingesetzte Implantat nicht mitwächst. Das Entfernen des Implantats erfordert eine weitere Operation am Organismus, die die bekannten physiologischen und psychologischen Nachteile zur Folge haben kann.

Eine Alternative zu Implantaten aus dauerhaft wirkenden Materialien sind resorbierbare Implantate. Resorbierbare Implantate sind solche Implantate, die aus einem resorbierbaren Material bestehen oder dieses Material umfassen. Resorbierbare Materialien werden auch als bioresorbierbare Materialien bezeichnet. Resorbierbare Materialien werden vom Körper durch Degradation abgebaut und müssen üblicherweise nicht aus diesem entfernt werden. Ein Vorteil hiervon ist, dass die im Vorherigen beschriebene weitere Operation am Organismus zur Entfernung des Implantats nicht erforderlich ist.

Resorbierbare Implantate aus Polymeren haben eine Vielzahl an Nachteilen. Einige Materialien führen zu Entzündungen und Fisteln, andere sind oftmals nicht stabil genug, um komplizierte oder größere Knochenbrüche zu heilen. Temporär oder als Dauerimplantate im Knochen eingesetzte Schrauben und Platten aus Stahl erfüllen zwar die Stabilitätskriterien, führen jedoch regelmäßig zu allergischen Reaktionen. Neben Stahlschrauben können auch Titanschrauben verwendet werden. Diese sind stabil und in der Regel allergisch unbedenklich, jedoch müssen diese, wie auch die Stahlimplantate, in den meisten Fällen wieder operativ entfernt werden.

Ein weiterer Nachteil von bekannten resorbierbaren Implantaten besteht darin, dass die Degradation bzw. der Abbau des Implantats nicht oder lediglich bedingt steuerbar ist. Resorbierbare Implantate degradieren in der Regel im Wesentlichen gleichmäßig. Hieraus folgt, dass das Implantat auch an unterschiedlichen Funktionsstellen gleichmäßig degradiert.

Bei resorbierbaren Implantaten, die derzeit häufig mittels Strangextrusion gefertigt werden, ist eine Steuerung der Degradationsgeschwindigkeit mittels einer Beschichtung des Implantats oder durch die Beimischung weiterer Legierungselemente, wie beispielsweise Seltener Erden, möglich. Hierdurch wird in der Regel jedoch lediglich eine globale Steuerung der Degradation möglich und keine abschnittsspezifische Degradation.

### Seite 3:

Die DE 10 2005 032 604 A1 beschreibt ein resorbierbares, in den Körper einsetzbares medizinisches Element, das eine nicht resorbierbare Beschichtung aufweist. Die US 7,879,367 B2 beschreibt einen Stent aus einem metallischen, resorbierbaren Material, das sich im Organismus durch Korrosion auflösen soll. Der Stent löst sich im Wesentlichen gleichmäßig auf.

Die US 2020/0121441 A1 beschreibt resorbierbare Implantate mit variabler Degradation, die eine Eindämmungsschicht enthalten. Der Abbau des Implantats wird mit der Eindämmungsschicht beeinflusst. Eine gezielte Steuerung des Abbaus ist nicht gelehrt.

Die WO 2019/072736 A1 offenbart ein Implantat zum zugfesten Verbinden zumindest zweier Teile eines gebrochenen Röhrenknochens.

Es ist daher eine Aufgabe der vorliegenden Erfindung, ein Implantat zum Implantieren in einen Organismus und ein Verfahren bereitzustellen, die einen oder mehrere der genannten Nachteile vermindern oder beseitigen. Insbesondere ist es eine Aufgabe der Erfindung, eine Lösung bereitzustellen, die ein verbessertes Implantat ermöglicht. Darüber hinaus ist es eine Aufgabe der Erfindung, eine Lösung bereitzustellen, die eine verbesserte Steuerung der Degradation eines Implantats ermöglicht.

Diese Aufgabe wird gelöst mit einem Implantat und einem Verfahren nach den Merkmalen der unabhängigen Patentansprüche. Weitere vorteilhafte Ausgestaltungen des Implantats und des Verfahrens sind in den jeweiligen abhängigen Patentansprüchen angegeben. Die in den Patentansprüchen einzeln aufgeführten Merkmale sind in beliebiger, technologisch sinnvoller Weise miteinander kombinierbar und können durch weitere Merkmale aus der Beschreibung ergänzt werden, wobei weitere Ausführungsvarianten der Erfindung aufgezeigt werden.

Das Implantat zum Implantieren in einen Organismus, insbesondere in den menschlichen Körper, umfasst einen ersten Implantatabschnitt, der eine erste Degradationsgeschwindigkeit aufweist, und einen zweiten Implantatabschnitt, der Seite 3a:
eine zweite Degradationsgeschwindigkeit aufweist, wobei die erste Degradationsgeschwindigkeit geringer als die zweite Degradationsgeschwindigkeit ist, und wobei das Implantat aus einem resorbierbaren Material besteht oder dieses umfasst.

Der Erfindung liegt die Erkenntnis zugrunde, dass sich im Wesentlichen global gleichförmig abbauende resorbierbare Implantate Nachteile aufweisen. Die Funktion eines Implantats kann insofern verbessert werden, dass einzelne Abschnitte des Implantats langsamer degradieren als andere Abschnitte. Beispielsweise kann bei einer geschraubten Knochenplatte der Abschnitt angrenzend an eine Schraube als erster Implantatabschnitt und der Bereich, der den Knochen stützt, als zweiter Implantatabschnitt ausgebildet sein. Infolgedessen degradiert der Stützabschnitt schneller als der Schraubabschnitt, sodass zunächst der Stützabschnitt abgebaut wird und bis zum vollständigen Abbau durch den Schraubabschnitt in seiner ursprünglichen Position gehalten wird. Hierdurch kann eine verbesserte Heilung ermöglicht werden.

Der erste Implantatabschnitt und der zweite Implantatabschnitt können in jeder Implantatausrichtung aneinander angrenzen. Der erste Implantatabschnitt und der zweite Implantatabschnitt sind vorzugsweise derart angeordnet, dass beide Implantatabschnitte einen Teil der Implantatoberfläche ausbilden. In dieser Ausführungsvariante sind der erste Implantatabschnitt und der zweite Implantatabschnitt insbesondere nebeneinander angeordnet.

Alternativ oder ergänzend können der erste Implantatabschnitt und der zweite Implantatabschnitt auch derart angeordnet sein, dass einer der Implantatabschnitte die Implantatoberfläche ausbildet und/oder der andere Implantatabschnitt von dem die Implantatoberfläche ausbildenden Implantatabschnitt umgeben, vorzugsweise vollständig umgeben, ist. Somit kann in besonders vorteilhafterweise ein Gradient der Degradationsgeschwindigkeit realisiert werden.

Das Implantat kann auch mehrere erste Implantatabschnitte und/oder mehrere zweite Implantatabschnitte aufweisen. Darüber hinaus kann das Implantat auch drei oder mehrere Implantatabschnitte mit jeweils unterschiedlichen Degradationsgeschwindigkeiten aufweisen.

Die Degradationsgeschwindigkeit ist die Geschwindigkeit, mit der das Implantat bzw. ein Implantatabschnitt innerhalb des bzw. vom Organismus abgebaut wird. Die Degradationsgeschwindigkeit kann vorzugsweise auch als

### Seite 5:

Abbaugeschwindigkeit bezeichnet werden. Die Degradationsgeschwindigkeit ist üblicherweise nicht linear, da diese zu Beginn hoch ist und mit der Zeit abnimmt.

Eine übliche Einheit zur Definition der Degradationsgeschwindigkeit ist Millimeter pro Zeiteinheit, beispielsweise Millimeter pro Tag, pro Woche, pro Monat und/oder pro Jahr. Darüber hinaus ist es bevorzugt, dass die Degradationsgeschwindigkeit als oberflächenbezogener Masseverlust je Zeiteinheit angegeben wird, beispielsweise mit der Einheit mg/cm²/Tag. Die Degradationsgeschwindigkeit als oberflächenbezogener Masseverlust je Zeiteinheit wird beispielsweise mittels der Formel Massenverlust dividiert durch die Oberfläche dividiert durch eine Zeiteinheit ermittelt.

Der erste Implantatabschnitt und der zweite Implantatabschnitt sind vorzugsweise im Wesentlichen aus dem gleichen resorbierbaren Material hergestellt. Das Implantat kann aus dem ersten Implantatabschnitt und dem zweiten Implantatabschnitt bestehen oder den ersten Implantatabschnitt und den zweiten Implantatabschnitt umfassen. Das Implantat ist vorzugsweise beschichtungsfrei ausgebildet. Das Implantat kann das resorbierbare Material umfassen oder aus diesem bestehen. Es ist insbesondere bevorzugt, dass das Implantat das resorbierbare Material zu mehr als 50 %, mehr als 75 %, mehr als 90 % oder mehr als 95 % umfasst.

Das Implantat zeichnet sich dadurch aus, dass der erste Implantatabschnitt eine erste Hohlraumstruktur mit einer ersten Fülldichte aufweist, und der zweite Implantatabschnitt eine zweite Hohlraumstruktur mit einer zweiten Fülldichte aufweist, wobei die erste Fülldichte höher als die zweite Fülldichte ist.

Die Fülldichte beschreibt insbesondere, wie groß das Verhältnis aus dem resorbierbaren Material und dem ausgebildeten Freiraum, der üblicherweise mit Luft gefüllt ist, ist. Die Fülldichte kann beispielsweise in Prozent zwischen 0 % und 100 % angegeben werden. Eine Fülldichte von 100 % bedeutet beispielsweise, dass der entsprechende Implantatabschnitt massiv ausgebildet ist. Unter einer

### Seite 5a

Hohlraumstruktur mit einer Fülldichte von 100 % ist vorzugsweise eine im Wesentlichen massive Struktur ohne Hohlräume zu verstehen.

Es kann bevorzugt sein, dass die erste Hohlraumstruktur eine erste Fülldichte von 100 % aufweist. Je geringer die Fülldichte ist, desto höher ist in der Regel die Degradationsgeschwindigkeit. Aus diesem Grund ist in dieser Fortbildung vorzugsweise vorgesehen, dass die erste Fülldichte höher als die zweite Fülldichte ist, sodass die erste Degradationsgeschwindigkeit geringer als die zweite Degradationsgeschwindigkeit ist.

Eine weitere bevorzugte Fortbildung des Implantats zeichnet sich dadurch aus, dass das Implantat einen Übergangsabschnitt aufweist, der zwischen dem ersten Implantatabschnitt und dem zweiten Implantatabschnitt angeordnet ist. Der Übergangsabschnitt weist vorzugsweise eine Hohlraumstruktur auf, die ferner vorzugsweise angrenzend an den ersten Implantatabschnitt eine im Wesentlichen der ersten Hohlraumstruktur entsprechende Hohlraumstruktur und/oder angrenzend an den zweiten Implantatabschnitt eine im Wesentlichen der zweiten Hohlraumstruktur entsprechende Hohlraumstruktur aufweist. Die Veränderung von der ersten Hohlraumstruktur hin zur zweiten Hohlraumstruktur des Übergangsabschnitts erfolgt insbesondere graduell.

Ein Implantat mit zwei oder mehr unterschiedlich ausgebildeten Hohlraumstrukturen ist einem Implantat aus einem Schaum überlegen, weil die Degradationsgeschwindigkeit in vorteilhafterweise steuerbar ist. Insbesondere die im Folgenden noch erläuterte geometrische Definiertheit der Hohlraumstrukturen ermöglicht eine gezielte Steuerung der Degradationsgeschwindigkeit, ohne dass auf unsichere und im medizinischen Bereich oftmals nicht ausreichende, statistische Methoden zur Verteilung der Hohlraumgröße zurückgegriffen werden muss.

In einer weiteren bevorzugten Ausführungsvariante des Implantats ist vorgesehen, dass die erste Fülldichte in Abhängigkeit der ersten Degradationsgeschwindigkeit und/oder die zweite Fülldichte in Abhängigkeit der zweiten Degradationsgeschwindigkeit gewählt ist. Beispielsweise kann aus medizinischer Perspektive eine bevorzugte erste Degradationsgeschwindigkeit und/oder zweite Degradationsgeschwindigkeit vorgegeben werden. Auf Basis dieser vorgegebenen Degradationsgeschwindigkeit kann das Implantat hinsichtlich seiner ersten und/oder zweiten Fülldichte derart ausgebildet werden, dass die medizinischen Anforderungen erfüllt werden.

Eine weitere bevorzugte Fortbildung des Implantats zeichnet sich dadurch aus, dass ein Fülldichtenverhältnis aus der ersten Fülldichte und der zweiten Fülldichte derart ausgewählt ist, dass die erste Degradationsgeschwindigkeit und die zweite Degradationsgeschwindigkeit ein vordefiniertes Geschwindigkeitsverhältnis ausbilden.

In einer weiteren bevorzugten Ausführungsvariante des Implantats ist vorgesehen, dass die erste Hohlraumstruktur und/oder die zweite Hohlraumstruktur geometrisch definiert ausgebildet ist bzw. sind. Geometrisch definiert kann beispielsweise bedeuten, dass diese ein geometrisches Muster aufweisen. Darüber hinaus kann geometrisch definiert bedeuten, dass die erste Hohlraumstruktur und/oder die zweite Hohlraumstruktur Figuren der Geometrie aufweisen. Weiterhin ist es bevorzugt, dass geometrisch definiert bedeutet, dass diese auf Gesetzen der Geometrie beruhend ausgebildet sind.

Eine weitere bevorzugte Fortbildung des Implantats zeichnet sich dadurch aus, dass Hohlräume der ersten Hohlraumstruktur und/oder der zweiten Hohlraumstruktur einen Querschnitt aufweisen. Der Querschnitt kann beliebig ausgebildet sein. Darüber hinaus kann der Querschnitt dreieckig, viereckig, pentagonal, hexagonal, polygonal und/oder gyroidförmig ausgebildet sein. Die Hohlraumstrukturen können Querschnitte mit einer, zwei, mehreren oder allen dieser Ausbildungen aufweisen.

Aus diesen möglichen Querschnitten, die einzeln oder in Kombination in der ersten Hohlraumstruktur und/oder der zweiten Hohlraumstruktur vorkommen können, ergeben sich entsprechend geformte dreidimensionale Hohlräume. Beispielsweise kann ein Hohlraum mit einem viereckigen Querschnitt in zwei Raumrichtungen eine kubische Gestalt annehmen. Die Anzahl der Hohlräume kann beliebig hoch sein sowie den individuellen Anforderungen und insbesondere an eine vordefinierte Degradationsgeschwindigkeit angepasst werden. Die Hohlräume sind vorzugsweise mittels Hohlraumwänden begrenzt und/oder voneinander abgegrenzt. Die Stärke der Hohlraumwände ist beliebig und kann innerhalb des Implantats variieren.

Eine weitere bevorzugte Ausführungsvariante des Implantats zeichnet sich dadurch aus, dass die erste Hohlraumstruktur und/oder die zweite Hohlraumstruktur eine Vielzahl an Hohlräumen aufweist, wobei die Hohlräume der ersten Hohlraumstruktur eine geringere Hohlraumgröße als die Hohlräume der zweiten Hohlraumstruktur aufweisen. Unter einer Hohlraumgröße kann beispielsweise ein Hohlraumvolumen, insbesondere ein durchschnittliches Hohlraumvolumen, verstanden werden. Darüber hinaus kann unter einer Hohlraumgröße eine Hohlraumabmessung, insbesondere eine durchschnittliche Hohlraumabmessung, verstanden werden, insbesondere in Richtung der größten Erstreckung des jeweiligen Hohlraums.

Dass die Hohlräume der ersten Hohlraumstruktur eine geringere Hohlraumgröße als die Hohlräume der zweiten Hohlraumstruktur aufweisen, kann auch bedeuten, dass die Hohlräume der ersten Hohlraumstruktur eine Hohlraumgröße von null haben und somit im Wesentlichen keine Hohlräume in der ersten Hohlraumstruktur vorhanden sind, sodass diese massiv ausgebildet ist.

Dass die Hohlräume der ersten Hohlraumstruktur eine geringere Hohlraumgröße als die Hohlräume der zweiten Hohlraumstruktur aufweisen, kann insbesondere bedeuten, dass mehr als 75 %, mehr als 90 % oder mehr als 95 % der Hohlräume der ersten Struktur kleiner als die Hohlräume der zweiten Hohlraumstruktur sind.

Eine weitere bevorzugte Fortbildung des Implantats zeichnet sich dadurch aus, dass die Hohlräume der ersten Hohlraumstruktur und/oder die Hohlräume der zweiten Hohlraumstruktur abgeschlossen zueinander ausgebildet sind. Abgeschlossen zueinander bedeutet insbesondere, dass diese zueinander abgetrennt sind. Abgeschlossen kann darüber hinaus bedeuten, dass im Wesentlichen keine fluidische Verbindung zwischen zwei benachbarten Hohlräumen vorhanden ist. Alternativ oder ergänzend können zwei benachbarte Hohlräume auch eine Verbindung, beispielsweise eine fluidische Verbindung, zueinander aufweisen.

In einer weiteren bevorzugten Ausführungsvariante des Implantats ist vorgesehen, dass der erste Implantatabschnitt und/oder der zweite Implantatabschnitt volumenförmig ausgebildet ist bzw. sind. Unter einer volumenförmigen Ausbildung ist insbesondere eine dreidimensionale Geometrie zu verstehen, die neben einer flächigen Ausbildung zusätzlich eine nicht zu vernachlässigende Dicke aufweist. Eine Beschichtung fällt üblicherweise nicht unter diese Definition.

Eine weitere bevorzugte Ausführungsvariante des Implantats zeichnet sich dadurch aus, dass das resorbierbare Material ein Metall ist oder ein Metall umfasst. Es ist insbesondere bevorzugt, dass das resorbierbare Material eine Magnesiumlegierung ist oder eine Magnesiumlegierung umfasst. Darüber hinaus kann das resorbierbare Material ein Polylactid sein oder umfassen.

Das aus einer Magnesiumlegierung hergestellte oder eine Magnesiumlegierung umfassende Implantat bietet mehrere Vorteile. Das Implantat ist metallisch und bietet somit eine hohe Stabilität, die in der Regel höher ist, als solche von Kunststoffen. Darüber hinaus werden solche Implantate im Körper vollständig umgebaut und bilden bei diesem Umbau die Grundlage für neue mineralisierte Knochensubstanz. Darüber hinaus sind solche Implantate ausreichend elastisch, um sogenannte Belastungsabschirmungen zu verhindern, sodass die Knochenbildung angeregt und die Heilung begünstigt wird.

In einer weiteren bevorzugten Ausführungsvariante des Implantats ist vorgesehen, dass das Implantat mit einem additiven Fertigungsverfahren hergestellt ist. Das additive Fertigungsverfahren ist oder umfasst vorzugsweise ein Materialextrusionsverfahren. Es ist ferner bevorzugt, dass das Materialextrusionsverfahren mit einem Sinterverfahren kombiniert wird, wobei das Sinterverfahren vorzugsweise nach dem Materialextrusionsverfahren eingesetzt wird.

In einer weiteren bevorzugten Ausführungsvariante des Implantats ist vorgesehen, dass der erste Implantatabschnitt in zumindest einer Implantatebene an den zweiten Implantatabschnitt angrenzt. Darüber hinaus ist es bevorzugt, dass der erste Implantatabschnitt von dem zweiten Implantatabschnitt in zumindest einer Implantatebene umschlossen ist.

Eine weitere bevorzugte Fortbildung des Implantats zeichnet sich dadurch aus, dass der erste Implantatabschnitt ein Befestigungsabschnitt und/oder der zweite Implantatabschnitt ein Strukturabschnitt ist. Alternativ oder ergänzend kann der erste Implantatabschnitt den Befestigungsabschnitt umfassen und/oder der zweite Implantatabschnitt den Strukturabschnitt umfassen. Der Befestigungsabschnitt weist vorzugsweise eine, zwei oder mehrere Durchgangsöffnungen auf. Der Befestigungsabschnitt ist vorzugsweise als ein Verschraubungsabschnitt ausgebildet. Der Strukturabschnitt ist ferner vorzugsweise als ein Verstärkungsabschnitt ausgebildet.

In einer weiteren bevorzugten Ausführungsvariante des Implantats ist vorgesehen, dass das Implantat eine Knochenplatte ist. Alternativ kann das Implantat auch eine Knochenschraube, eine Wundklammer, ein Stent, eine Vorrichtung zur Implantatfixierung und/oder ein Trägermaterial für Tissue Engineering sein. Es ist insbesondere bevorzugt, dass die Knochenplatte oder eine der im Vorherigen genannten Ausbildungen des Implantats den Befestigungsabschnitt und den Strukturabschnitt aufweisen. Somit kann die Knochenplatte mittels des Befestigungsabschnitts an einem Knochen verschraubt werden. Der Strukturabschnitt dient der Abstützung des Knochens.

In einer weiteren bevorzugten Ausführungsvariante des Implantats ist vorgesehen, dass mindestens ein Hohlraum ein Arzneimittel enthält. Diese Ausführungsform hat den Vorteil, dass das Arzneimittel bei der Degradation des Implantats freigesetzt wird, sodass über unterschiedliche Degradationsgeschwindigkeiten die Geschwindigkeit der Arzneimittelabgabe steuerbar ist.

Darüber hinaus ist es bevorzugt, dass das Implantat eine Beschichtung aufweist. Die Beschichtung besteht vorzugsweise aus einem resorbierbaren Material oder umfasst dieses. Die Beschichtung kann im Wesentlichen vollständig oder teilweise eine Implantatoberfläche ausbilden.

Gemäß einem weiteren Aspekt wird die eingangs genannte Aufgabe gelöst durch ein Verfahren zur Herstellung eines Implantats, insbesondere eines Implantats nach einer der im Vorherigen beschriebenen Ausführungsvarianten, umfassend die Schritte: Erzeugen eines ersten Implantatabschnitts aus oder mit einem resorbierbaren Material, der eine erste Degradationsgeschwindigkeit aufweist, und Erzeugen eines zweiten Implantatabschnitts aus oder mit einem resorbierbaren Material, der eine zweite Degradationsgeschwindigkeit aufweist, wobei die erste Degradationsgeschwindigkeit geringer als die zweite Degradationsgeschwindigkeit ist.

Eine bevorzugte Ausführungsvariante des Verfahrens sieht vor, dass der erste Implantatabschnitt und/oder der zweite Implantatabschnitt mit einer Hohlraumstruktur erzeugt wird, wobei der erste Implantatabschnitt eine erste Fülldichte und/oder der zweite Implantatabschnitt eine zweite Fülldichte aufweist, wobei vorzugsweise die erste Fülldichte höher als die zweite Fülldichte ist.

Darüber hinaus ist es bevorzugt, dass der erste Implantatabschnitt und/oder der zweite Implantatabschnitt mit einem additiven Fertigungsverfahren erzeugt wird.

Das Verfahren und seine möglichen Fortbildungen weisen Merkmale bzw. Verfahrensschritte auf, die sie insbesondere dafür geeignet machen, für das Implantat und seine Fortbildungen verwendet zu werden. Für weitere Vorteile, Ausführungsvarianten und Ausführungsdetails des Verfahrens und seiner möglichen Fortbildungen wird auch auf die zuvor erfolgte Beschreibung zu den entsprechenden Merkmalen und Fortbildungen des Implantats verwiesen.

Bevorzugte Ausführungsbeispiele werden exemplarisch anhand der beiliegenden Figuren erläutert. Es zeigen:
- Figur 1:: eine schematische, zweidimensionale Ansicht einer beispielhaften Ausführungsform eines Implantats;
- Figur 2:: eine weitere schematische, zweidimensionale Ansicht einer beispielhaften Ausführungsform eines Implantats; und
- Figur 3:: ein schematisches Verfahren.

In den Figuren sind gleiche oder im Wesentlichen funktionsgleiche bzw. -ähnliche Elemente mit den gleichen Bezugszeichen bezeichnet.

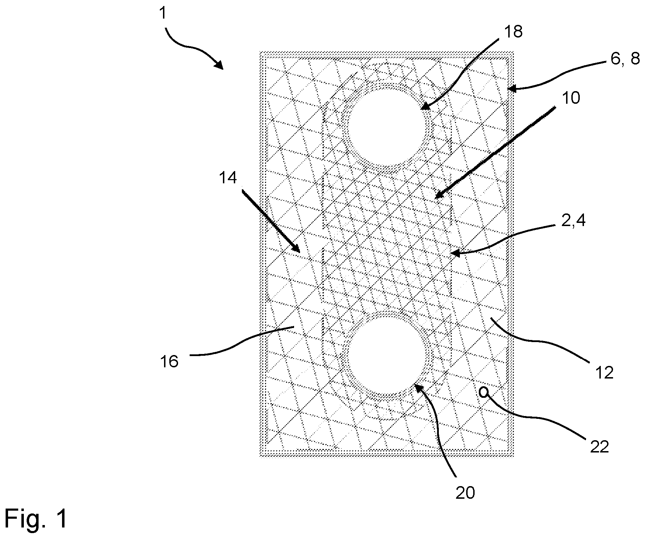

Figur 1 zeigt ein Implantat 1 zum Implantieren in einen Organismus, insbesondere in den menschlichen Körper. Das Implantat 1 umfasst einen ersten Implantatabschnitt 2, der als Verschraubungsabschnitt 4 ausgebildet ist. Darüber hinaus umfasst das Implantat 1 einen zweiten Implantatabschnitt 6, der als Verstärkungsabschnitt 8 ausgebildet ist.

Der erste Implantatabschnitt 2 weist eine erste Degradationsgeschwindigkeit und der zweite Implantatabschnitt 6 weist eine zweite Degradationsgeschwindigkeit auf. Das Implantat 1 besteht aus einem resorbierbaren Material, das eine Magnesiumlegierung ist. Das Implantat 1 kann auch ein anderes geeignetes resorbierbares Material aufweisen oder aus diesem bestehen.

Der erste Implantatabschnitt 2 weist eine erste Hohlraumstruktur 10 auf. Die erste Hohlraumstruktur 10 weist darüber hinaus eine erste Fülldichte auf, wobei zu erkennen ist, dass die erste Hohlraumstruktur 10 vergleichsweise engmaschig ausgebildet ist. Der zweite Implantatabschnitt weist eine zweite Hohlraumstruktur 14 auf, die eine zweite Fülldichte aufweist. Es ist gezeigt, dass die zweite Hohlraumstruktur 14 weniger engmaschig ausgebildet ist als die erste Hohlraumstruktur 10.

Die Hohlraumstrukturen 10, 14 weisen jeweils Hohlräume 12, 16 auf. Der erste Implantatabschnitt 2 weist darüber hinaus eine erste Verschraubungsöffnung 18 und eine zweite Verschraubungsöffnung 20 auf. Ferner ist in einem der Hohlräume 12 des zweiten Implantatabschnitts 6 ein Arzneimittel 22 eingeschlossen.

Die erste Hohlraumstruktur 10 und die zweite Hohlraumstruktur 14 sind geometrisch definiert ausgebildet. Die Hohlräume der ersten Hohlraumstruktur 10 und der zweiten Hohlraumstruktur 14 weisen einen Querschnitt auf, der dreieckig ausgebildet ist. Die Hohlräume können beispielsweise pyramidenförmig ausgebildet sein.

Die erste Hohlraumstruktur 10 und die zweite Hohlraumstruktur 14 weisen eine Vielzahl an Hohlräumen 12, 16 auf, wobei die Hohlräume der ersten Hohlraumstruktur 10 eine geringere Hohlraumgröße als die Hohlräume der zweiten Hohlraumstruktur 14 auf. Die erste Degradationsgeschwindigkeit ist daher geringer als die zweite Degradationsgeschwindigkeit.

Der Querschnitt der Hohlräume 12 der zweiten Hohlraumstruktur weist circa eine viermal so große Fläche auf wie der Querschnitt der Hohlräume 12 der ersten Hohlraumstruktur 10. Die Hohlräume 12, 16 der Hohlraumstrukturen 10, 14 sind abgeschlossen zueinander ausgebildet.

Der erste Implantatabschnitt 2 grenzt an den zweiten Implantatabschnitt 6 an und ist von diesem in der Ansichtsebene umschlossen. Das Implantat 1 ist mit einem additiven Fertigungsverfahren hergestellt.

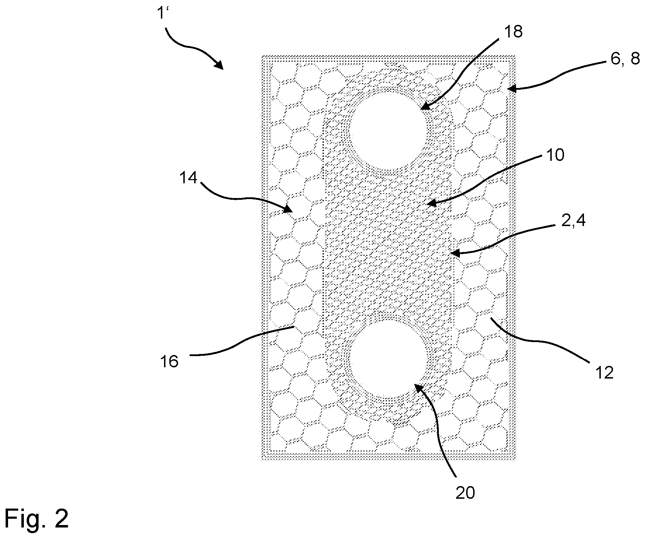

Figur 2 zeigt eine weitere Ausführungsvariante eines Implantats 1'. Das Implantat 1' ist im Wesentlichen gleich aufgebaut wie das Implantat 1, wobei sich der erste Implantatabschnitt 2 und der zweite Implantatabschnitt 6 von denen in Figur 1 derart unterscheiden, dass deren Querschnitte hexagonal bzw. wabenförmig ausgebildet sind.

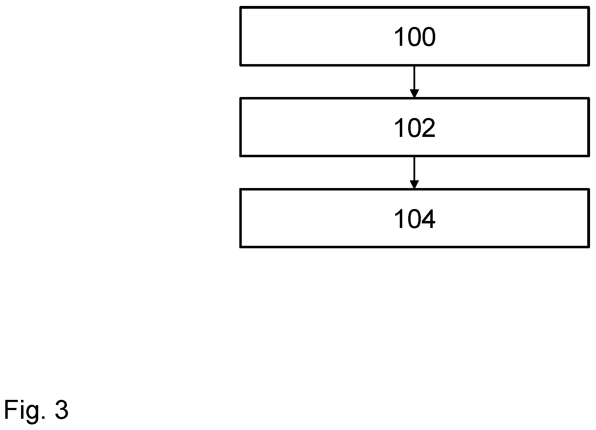

Figur 3 zeigt ein schematisches Verfahren. Das Verfahren umfasst die Schritte 100, 102, 104. In Schritt 100 wird ein resorbierbares Material bereitgestellt. In Schritt 102 wird ein erster Implantatabschnitt 2 aus dem resorbierbaren Material erzeugt. Der erste Implantatabschnitt 2 weist eine erste Degradationsgeschwindigkeit auf, die im Wesentlichen aufgrund der erzeugten Hohlraumstruktur 10 bewirkt wird. In Schritt 104 wird ein zweiter Implantatabschnitt 6 aus dem resorbierbaren Material erzeugt, der eine zweite Degradationsgeschwindigkeit aufweist, die im Wesentlichen aufgrund der erzeugten Hohlraumstruktur 14 bewirkt wird.

Die erste Degradationsgeschwindigkeit ist geringer als die zweite Degradationsgeschwindigkeit. Dies wird insbesondere dadurch erreicht, dass die Fülldichte der zweiten Hohlraumstruktur 14 geringer ist als die Fülldichte der ersten Hohlraumstruktur 10. Die Schritte 102 und 104 können auch gleichzeitig durchgeführt werden. Darüber hinaus kann auch der Schritt 104 vor dem Schritt 102 durchgeführt werden.

Es ist bevorzugt, dass der erste Implantatabschnitt 2 und der zweite Implantatabschnitt 6 mit der im Vorherigen genannten Hohlraumstruktur 10, 14 erzeugt wird, wobei der erste Implantatabschnitt 2 eine erste Fülldichte und der zweite Implantatabschnitt 6 eine zweite Fülldichte aufweist. Die erste Fülldichte ist geringer als die zweite Fülldichte.

Das Implantat 1 mit zwei Implantatabschnitten 2, 6 mit unterschiedlichen Degradationsgeschwindigkeiten, ermöglicht komplexe Implantate und neue Behandlungsmöglichkeiten, zum Beispiel von Knochenbrüchen. Je nachdem an welchem Abschnitt das Implantat länger Belastungen aufzunehmen hat, kann eine höhere oder niedrigere Degradationsgeschwindigkeit vorgesehen werden. Insbesondere ist es somit möglich, die Degradation abschnittsspezifisch zu steuern und somit der Medizin einen größeren Handlungsspielraum zu ermöglichen.

### BEZUGSZEICHEN

- 1, 1': Implantat
- 2: erster Implantatabschnitt
- 4: Verschraubungsabschnitt
- 6: zweiter Implantatabschnitt
- 8: Verstärkungsabschnitt
- 10: erste Hohlraumstruktur
- 12: Hohlraum
- 14: zweite Hohlraumstruktur
- 16: Hohlraum
- 18: erste Verschraubungsöffnung
- 20: zweite Verschraubungsöffnung
- 22: Arzneimittel

## Patentansprüche

1. Implantat (1, 1') zum Implantieren in einen Organismus, insbesondere in den menschlichen Körper, umfassend
- einen ersten Implantatabschnitt (2), der eine erste Degradationsgeschwindigkeit aufweist, und
- einen zweiten Implantatabschnitt (6), der eine zweite Degradationsgeschwindigkeit aufweist,
- wobei die erste Degradationsgeschwindigkeit geringer als die zweite Degradationsgeschwindigkeit ist, und
- wobei das Implantat (1, 1') aus einem resorbierbaren Material besteht oder dieses umfasst,
- **dadurch gekennzeichnet, dass** der erste Implantatabschnitt (2) eine erste Hohlraumstruktur (10) mit einer ersten Fülldichte aufweist und der zweite Implantatabschnitt (6) eine zweite Hohlraumstruktur (14) mit einer zweiten Fülldichte aufweist, wobei die erste Fülldichte höher als die zweite Fülldichte ist.

2. Implantat (1, 1') nach dem vorherigen Anspruch 1, wobei
- die erste Fülldichte in Abhängigkeit der ersten Degradationsgeschwindigkeit und/oder die zweite Fülldichte in Abhängigkeit der zweiten Degradationsgeschwindigkeit gewählt ist, und/oder
- ein Fülldichtenverhältnis aus der ersten Fülldichte und der zweiten Fülldichte derart ausgewählt ist, dass die erste Degradationsgeschwindigkeit und die zweite Degradationsgeschwindigkeit ein vordefiniertes Geschwindigkeitsverhältnis ausbilden.

3. Implantat (1, 1') nach einem der vorherigen Ansprüche, wobei
- die erste Hohlraumstruktur (10) und/oder die zweite Hohlraumstruktur (14) geometrisch definiert ausgebildet ist bzw. sind, und/oder
- Hohlräume der ersten Hohlraumstruktur (10) und/oder der zweiten Hohlraumstruktur (14) einen Querschnitt aufweisen, der dreieckig, viereckig, pentagonal, hexagonal, polygonal und/oder gyroidförmig ausgebildet ist.

4. Implantat (1, 1') nach einem der vorherigen Ansprüche, wobei
- die erste Hohlraumstruktur (10) und/oder die zweite Hohlraumstruktur (14) eine Vielzahl an Hohlräumen (12, 16) aufweist, wobei die Hohlräume (12) der ersten Hohlraumstruktur (10) eine geringere Hohlraumgröße als die Hohlräume (16) der zweiten Hohlraumstruktur (14) aufweisen.

5. Implantat (1, 1') nach einem der vorherigen Ansprüche, wobei
- die Hohlräume (12) der ersten Hohlraumstruktur (10) und/oder die Hohlräume (16) der zweiten Hohlraumstruktur (14) abgeschlossen zueinander ausgebildet sind.

6. Implantat (1, 1') nach einem der vorherigen Ansprüche, wobei
- der erste Implantatabschnitt (2) und/oder der zweite Implantatabschnitt (6) volumenförmig ausgebildet ist bzw. sind.

7. Implantat (1, 1') nach einem der vorherigen Ansprüche, wobei das resorbierbare Material ein Metall, insbesondere eine Magnesiumlegierung, ist oder ein Metall, insbesondere eine Magnesiumlegierung, umfasst.

8. Implantat (1, 1') nach einem der vorherigen Ansprüche, wobei das Implantat (1, 1') mit einem additiven Fertigungsverfahren hergestellt ist.

9. Implantat (1, 1') nach einem der vorherigen Ansprüche, wobei der erste Implantatabschnitt (2) in zumindest einer Implantatebene an den zweiten Implantatabschnitt (6) angrenzt und vorzugsweise von diesem umschlossen ist.

10. Implantat (1, 1') nach einem der vorherigen Ansprüche, wobei der erste Implantatabschnitt (2) ein Befestigungsabschnitt, insbesondere ein Verschraubungsabschnitt (4), und/oder der zweite Implantatabschnitt (6) ein Strukturabschnitt, insbesondere ein Verstärkungsabschnitt (8) zum Verstärken eines Knochens, ist und/oder umfasst.

11. Implantat (1, 1') nach einem der vorherigen Ansprüche, wobei das Implantat (1, 1') eine Knochenplatte ist.

12. Implantat (1, 1') nach einem der vorherigen Ansprüche, wobei mindestens ein Hohlraum (12, 16) ein Arzneimittel enthält.

13. Verfahren zur Herstellung eines Implantats (1, 1'), insbesondere eines Implantats (1, 1') nach einem der vorherigen Ansprüche 1-12, umfassend die Schritte:
- Erzeugen eines ersten Implantatabschnitts (2) aus oder mit einem resorbierbaren Material, der eine erste Degradationsgeschwindigkeit aufweist, und
- Erzeugen eines zweiten Implantatabschnitts (6) aus oder mit einem resorbierbaren Material, der eine zweite Degradationsgeschwindigkeit aufweist,
- wobei die erste Degradationsgeschwindigkeit geringer als die zweite Degradationsgeschwindigkeit ist,
- **dadurch gekennzeichnet, dass** der erste Implantatabschnitt (2) und der zweite Implantatabschnitt (6) mit einer Hohlraumstruktur (10, 14) erzeugt werden, wobei der erste Implantatabschnitt (2) eine erste Fülldichte und der zweite Implantatabschnitt (6) eine zweite Fülldichte aufweist, wobei die erste Fülldichte höher als die zweite Fülldichte ist.

14. Verfahren nach dem vorherigen Anspruch 13, wobei
- der erste Implantatabschnitt (2) und/oder der zweite Implantatabschnitt (6) mit einem additiven Fertigungsverfahren erzeugt wird.

## Claims

1. Implant (1, 1') for implantation in an organism, in particular in the human body, comprising
- a first implant section (2) which has a first degradation speed, and
- a second implant section (6) which has a second degradation speed,
- wherein the first degradation rate is less than the second degradation rate, and
- wherein the implant (1, 1') consists of or comprises a resorbable material,
- **characterized in that** the first implant portion (2) has a first cavity structure (10) with a first filling density and the second implant portion (6) has a second cavity structure (14) with a second filling density, wherein the first filling density is higher than the second filling density.

2. Implant (1, 1') according to the preceding claim 1, wherein
- the first filling density is selected as a function of the first degradation rate and/or the second filling density is selected as a function of the second degradation rate, and/or
- a filling density ratio is selected from the first filling density and the second filling density in such a way that the first degradation speed and the second degradation speed form a predefined speed ratio.

3. Implant (1, 1') according to any one of the preceding claims, wherein
- the first cavity structure (10) and/or the second cavity structure (14) is or are configured in a geometrically defined manner, and/or
- cavities of the first cavity structure (10) and/or the second cavity structure (14) have a cross-section that is configured triangular, quadrangular, pentagonal, hexagonal, polygonal and/or gyroidal.

4. Implant (1, 1') according to any one of the preceding claims, wherein
- the first cavity structure (10) and/or the second cavity structure (14) has a plurality of cavities (12, 16), wherein the cavities (12) of the first cavity structure (10) have a smaller cavity size than the cavities (16) of the second cavity structure (14).

5. Implant (1, 1') according to any one of the preceding claims, wherein
- the cavities (12) of the first cavity structure (10) and/or the cavities (16) of the second cavity structure (14) are configured to be closed with respect to each other.

6. Implant (1, 1') according to any one of the preceding claims, wherein
- the first implant section (2) and/or the second implant section (6) is or are configured in the form of a volume.

7. Implant (1, 1') according to any one of the preceding claims, wherein the resorbable material is a metal, in particular a magnesium alloy, or comprises a metal, in particular a magnesium alloy.

8. Implant (1, 1') according to any one of the preceding claims, wherein the implant (1, 1') is produced using an additive manufacturing process.

9. Implant (1, 1') according to any one of the preceding claims, wherein the first implant portion (2) is adjacent to and preferably enclosed by the second implant portion (6) in at least one implant plane.

10. Implant (1, 1') according to any one of the preceding claims, wherein the first implant section (2) is and/or comprises a fastening section, in particular a screwing section (4), and/or the second implant section (6) is and/or comprises a structural section, in particular a reinforcing section (8) for reinforcing a bone.

11. Implant (1, 1') according to any one of the preceding claims, wherein the implant (1, 1') is a bone plate.

12. Implant (1, 1') according to any one of the preceding claims, wherein at least one cavity (12, 16) contains a drug.

13. Method for producing an implant (1, 1'), in particular an implant (1, 1') according to any one of the preceding claims 1-12, comprising the steps of:
- Producing a first implant portion (2) from or with a resorbable material having a first degradation rate, and
- creating a second implant portion (6) from or with a resorbable material having a second degradation rate,
- wherein the first degradation rate is lower than the second degradation rate,
- **characterized in that** the first implant portion (2) and the second implant portion (6) are produced with a cavity structure (10, 14), wherein the first implant portion (2) has a first filling density and the second implant portion (6) has a second filling density, wherein the first filling density is higher than the second filling density.

14. Method according to the preceding claim 13, wherein
- the first implant section (2) and/or the second implant section (6) is produced using an additive manufacturing process.

## Revendications

1. Implant (1, 1') destiné à être implanté dans un organisme, en particulier dans le corps humain, comprenant
- une première section d'implant (2) qui présente une première vitesse de dégradation, et
- une deuxième section d'implant (6) qui présente une deuxième vitesse de dégradation,
- dans lequel la première vitesse de dégradation est inférieure à la deuxième vitesse de dégradation, et
- l'implant (1, 1') est constitué ou comprend un matériau résorbable,
- **caractérisé en ce que** la première partie d'implant (2) présente une première structure alvéolaire (10) avec une première densité de remplissage et la deuxième partie d'implant (6) présente une deuxième structure alvéolaire (14) avec une deuxième densité de remplissage, la première densité de remplissage étant supérieure à la deuxième densité de remplissage.

2. Implant (1, 1') selon la revendication 1, dans lequel
- la première densité de remplissage est choisie en fonction de la première vitesse de dégradation et/ou la deuxième densité de remplissage est choisie en fonction de la deuxième vitesse de dégradation, et/ou
- un rapport de densité de remplissage est choisi entre la première densité de remplissage et la deuxième densité de remplissage de telle sorte que la première vitesse de dégradation et la deuxième vitesse de dégradation forment un rapport de vitesse prédéfini.

3. Implant (1, 1') selon l'une quelconque des revendications précédentes, dans lequel
- la première structure creuse (10) et/ou la deuxième structure creuse (14) est ou sont configurées de manière géométriquement définie, et/ou
- les cavités de la première structure de cavités (10) et/ou de la deuxième structure de cavités (14) ont une section transversale configurée de manière triangulaire, quadrangulaire, pentagonale, hexagonale, polygonale et/ou gyroïdale.

4. Implant (1, 1') selon l'une quelconque des revendications précédentes, dans lequel
- la première structure creuse (10) et/ou la deuxième structure creuse (14) comporte une pluralité de cavités (12, 16), les cavités (12) de la première structure creuse (10) ayant une taille plus petite que les cavités (16) de la deuxième structure creuse (14).

5. Implant (1, 1') selon l'une quelconque des revendications précédentes, dans lequel
- les cavités (12) de la première structure de cavités (10) et/ou les cavités (16) de la deuxième structure de cavités (14) sont configurées pour être fermées les unes par rapport aux autres.

6. Implant (1, 1') selon l'une quelconque des revendications précédentes, dans lequel
- la première section d'implant (2) et/ou la deuxième section d'implant (6) est ou sont configurées sous la forme d'un volume.

7. Implant (1, 1') selon l'une quelconque des revendications précédentes, dans lequel le matériau résorbable est un métal, en particulier un alliage de magnésium, ou comprend un métal, en particulier un alliage de magnésium.

8. Implant (1, 1') selon l'une quelconque des revendications précédentes, dans lequel l'implant (1, 1') est fabriqué selon un procédé de fabrication additive.

9. Implant (1, 1') selon l'une quelconque des revendications précédentes, dans lequel la première partie d'implant (2) est adjacente à la deuxième partie d'implant (6) et de préférence entourée par celle-ci dans au moins un plan d'implant.

10. Implant (1, 1') selon l'une quelconque des revendications précédentes, dans lequel la première section d'implant (2) est et/ou comprend une section de fixation, en particulier une section de vissage (4), et/ou la deuxième section d'implant (6) est et/ou comprend une section structurelle, en particulier une section de renfort (8) pour renforcer un os.

11. Implant (1, 1') selon l'une quelconque des revendications précédentes, dans lequel l'implant (1, 1') est une plaque osseuse.

12. Implant (1, 1') selon l'une quelconque des revendications précédentes, dans lequel au moins une cavité (12, 16) contient un médicament.

13. Procédé de fabrication d'un implant (1, 1'), en particulier d'un implant (1, 1') selon l'une quelconque des revendications 1 à 12, comprenant les étapes consistant à :
- fabriquer une première partie d'implant (2) à partir d'un matériau résorbable ayant une première vitesse de dégradation, et
- création d'une deuxième partie d'implant (6) à partir d'un matériau résorbable ayant un deuxième taux de dégradation, ou avec un tel matériau,
- dans lequel le premier taux de dégradation est inférieur au deuxième taux de dégradation,
- **caractérisé en ce que** la première partie d'implant (2) et la deuxième partie d'implant (6) sont produites avec une structure creuse (10, 14), dans laquelle la première partie d'implant (2) a une première densité de remplissage et la deuxième partie d'implant (6) a une deuxième densité de remplissage, la première densité de remplissage étant supérieure à la deuxième densité de remplissage.

14. Procédé selon la revendication 13 précédente, dans lequel
- la première partie d'implant (2) et/ou la deuxième partie d'implant (6) est fabriquée à l'aide d'un procédé de fabrication additive.
